# EUROPEAN PATENT SPECIFICATION

(11) **EP 3 185 855 B1**
(45) Date of publication and mention of the grant of the patent: **07.09.2022**
(21) Application number: 15739632.6
(22) Date of filing: 23.07.2015
(51) Int. Cl.: A61K 9/20, A61P 29/00, A61K 31/192, A61K 31/522, A61P 25/04

(54) **FILM COATED TABLET FOR THE TREATMENT OF ACUTE PAIN**
FILMTABLETTE ZUR BEHANDLUNG VON AKUTEM SCHMERZ
COMPRIMÉS PELLICULÉS POUR LE TRAITEMENT DE LA DOULEUR AIGUË

(30) Priority: 28.08.2014 EP 14002975
(43) Date of publication of application: 05.07.2017
(73) Proprietor: Sanofi-Aventis Deutschland GmbH, 65926 Frankfurt am Main (DE); Sanofi-Aventis de Mexico SA de CV, 04000 Mexico D.F. (MX)
(72) Inventor: SAUERLAND, Sandra, 55216 Ingelheim am Rhein (DE); FUERST, Thomas, 55216 Ingelheim am Rhein (DE); HEGEWISCH, Alberto, 16090 Mexico, D.F. (MX); WEISER, Thomas, 55216 Ingelheim am Rhein (DE)
(74) Representative: Weiss, Wolfgang
(86) International application number: PCT/EP2015/066884
(87) International publication number: WO 2016/030091

(56) References cited:
- CA-C- 1 336 687
- US-A1- 2007 077 297
- DATABASE WPI Week 200574 2005 Thomson Scientific, London, GB; AN 2005-719553 XP002744685, -& JP 2005 289906 A (ZERIA SHINYAKU KOGYO KK) 20 October 2005 (2005-10-20)
- DOOLEY ET AL: "Caffeine as an Adjuvant to Ibuprofen in Treating Childhood Headaches", PEDIATRIC NEUROLOGY, ELSEVIER SCIENCE, NL, vol. 37, no. 1, 10 July 2007 (2007-07-10), pages 42-46, XP022145301, ISSN: 0887-8994, DOI: 10.1016/J.PEDIATRNEUROL.2007.02.016

## Description

### CROSS REFERENCE TO RELATED APPLICATIONS

This application claims the benefit of priority to European Patent Application No. 14002975.2, filed on August 28, 2014.

### BACKGROUND OF THE INVENTION

### 1. TECHNICAL AREA

The invention relates to a film coated tablet as defined in the claims for the treatment of acute pain, containing 400 mg of ibuprofen, 100 mg of caffeine, 50 to 100 mg of one or more disintegrating agents.

### 2. PRIOR ART

Ibuprofen or (+/-) 2-(p-isobutylphenyl)-propionic acid of formula 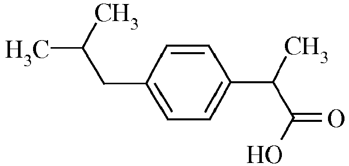 is known since long as an NSAID medication with analgesic and antipyretic activity. Caffeine or 3,7-dihydro-1,3,7-trimethyl-1H-purine-2,6-dione of formula 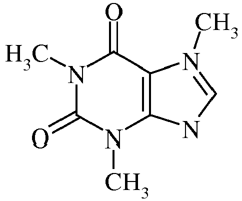 has long been used alone or together with other active substances for the treatment of acute pain.

US patent 4, 420,483 suggests the use of caffeine for accelerating the analgesic and anti-inflammatory activity of ibuprofen.

In European patent application EP 1 518 551 A1, solid pharmaceutical administration forms are suggested, which in addition to caffeine in uncoated form with a mean particle size of about 70 to 600 µm contain a headache relieving agent, including ibuprofen

The therapeutic combination of 400 mg ibuprofen and 100 mg caffeine is known from DOOLEY et al.: "Caffeine as an Adjuvant to Ibuprofen in Treating Childhood Headaches", PEDIATRIC NEUROLOGY, vol. 37, no. 1, pages 42-46. This document discloses oral administration of separate capsules comprising ibuprofen and caffeine.

The present invention was based on the aim of providing a pharmaceutical composition for oral administration which allows to treat acute pain of mild to severe intensity rapidly and alleviates pain for more than six hours.

Surprisingly, within the framework of extensive clinical trials it was shown that a film coated tablet as defined in the claims that contains 400 mg of ibuprofen, 100 mg of caffeine, 50 to 100 mg of one or more disintegrating agents is excellently suited for treating acute pain within a short time and with a long duration of action.

### BRIEF SUMMARY OF THE INVENTION

The present invention relates to a film coated tablet wherein the core of the tablet consists of 400 mg of ibuprofen, 100 mg of caffeine, 50 to 100 mg of one or more disintegrating agents and one or more further components selected from the group of carriers, flow regulating agents and lubricants,characterized in that
- the core of the tablet is obtainable by direct dry compression of all the components and consists of 90 to 98 % by weight of ibuprofen, caffeine and one or more disintegrating agents and of 2 to 10% by weight of one or more further components selected from the group of carriers, flow regulating agents and lubricants;
- the disintegrating agents are selected from microcrystalline cellulose and a combination of microcrystalline cellulose and croscarmellose;
- the carrier is selected from the group consisting of lactose, cellulose, saccharose, polyethylene glycol and polyethylene oxide (PEO).

Another aspect of the present invention is a process for the manufacture of a film coated tablet in accordance with any one of the claims 1 to 3, wherein the components as defined in said claims are dry compressed and coated with a coating.

A further aspect of the invention is the tablet according to one of the claims 1-3 for use in the treatment of acute pain.

### BRIEF DESCRIPTION OF THE DRAWINGS

Figures 1 and 2 show bar graphs of SPRID0-8h and SPRID0-2h, \respectively. The fix dose combination (FDC) of ibuprofen and caffeine ("Ibup/Caff') is 30-50% more effective than 400 mg ibuprofen alone. Values are shown as means + SEM adjusted for baseline pain intensity as measured on the 4-point verbal rating scale VRS.
Figure 3 shows adjusted means for pain intensity difference over time.
Figure 4 shows Kaplan-Meier estimates over time for time to perceptible pain relief.
Figure 5 shows Kaplan-Meier estimates over time for time to meaningful pain relief.

### DETAILED DESCRIPTION OF THE INVENTION

The term "ibuprofen", as used herein above and herein below, comprises the active substance 2-(p-isobutylphenyl)-propionic acid in any form, i.e., as a salt, as a free acid, as an enantiomer or enantiomer mixture; the racemate of the free acid is preferred.

The term "caffeine", as used herein above and herein below, comprises natural and synthetic 3,7-dihydro-1,3,7-trimethyl-1H-purine-2,6-dione in any form as an amorphous powder or in the form of crystals with a certain particle size distributions.

The film coated tablet may be coated with conventional coating materials. The core of the tablet is defined in the claims and contains conventional additives and excipients that are useful with film coated tablets. The lubricants can be selected from magnesium stearate, stearic acid, talc, vegetable oils and waxes.

The core of the tablet is produced by dry direct pressing methods.

The film coated tablet according to the invention has the following characteristics:
(a) the disintegrating agents are selected from the group consisting of microcrystalline cellulose and a combination of microcrystalline cellulose and croscarmellose; preferably, wherein a combination of microcrystalline cellulose and sodium croscarmellose is used as the disintegrating agent, in particular wherein the weight ratio of microcrystalline cellulose to sodium croscarmellose is 4-5 to 1;
(b) the carrier is selected from the group consisting of lactose, cellulose, saccharose, polyethylene glycol and polyethylene oxide (PEO); in particular, wherein the carrier is a mixture of different types of cellulose;
(c) the core of the tablet consist of 90 to 98 % by weight of ibuprofen, caffeine and the disintegrating agents as defined above and of 2 to 10% by weight of one or more further components selected from the group of carriers as defined above, flow regulating agents and lubricants; in particular wherein the weight ratio between caffeine and one or more disintegrants is 1.0 to 0.1-0.9, preferably, wherein the weight ratio between caffeine and sodium croscarmellose is 5-10 to 1;
(d) it is used for the treatment of acute pain, preferably for the treatment of acute dental or jaw pain or for the treatment of acute headaches; in particular for the treatment of acute dental or jaw pain caused by dental extraction
(e) its pain-relieving effect begins within 15 to 180 minutes after administration in at least 15% of patients evaluated according to a Kaplan-Meyer analysis ( e.g. Kaplan, E. L.; Meier, P. (1958). "Nonparametric estimation from incomplete observations". J. Amer. Statist. Assn. 53 (282): 457-481. JSTOR 2281868);
(f) its pain-relieving effect lasts for at least 6 to 8 hours after administration in at least 60% of patients evaluated according to a Kaplan-Meyer analysis;
(g) it achieves a reduction of ≥5.9 on the numerical pain rating scale (NPRS) ranging from 0 to 10;
(h) it contains
   10 to 50 mg of the carrier material as defined in the claims,
   50 to 90 mg, of the disintegrating agents as defined in the claims, in particular 62 to 88 mg of two disintegrating agents as defined in the claims,
   1 to 5 mg of one or more flow regulating agents, especially a colloidal silica, for example an Aerosil^{®} product from the firm of Evonik Industries AG, Rodenbacher Chaussee 4, 63457 Hanau-Wolfgang, and
   1 to 5 mg of one or more lubricants.

The following non-limiting examples will further illustrate the invention.

### Example 1

A film coated tablet is prepared containing:

| No. | Constituent | Quantity [mg] |
|---|---|---|
| 1 | Ibuprofen | 400 |
| 2 | Caffeine | 100 |
| 3 | Microcrystalline cellulose | 70.6 |
| 4 | Sodium croscarmellose | 15 |
| 5 | Cellulose | 24 |
| 6 | Colloidal silica | 2.6 |
| 7 | Magnesium stearate | 2.6 |
| 8 | Film Aqua Polish P | 15 |
| 9 | Water | 45 |

Components 1 to 7 are mixed together and pressed into a tablet. Subsequently the tablet is coated with constituents 8 and 9.

### Example 2

Clinical trials are performed in patients using the film coated tablets produced according to example 1 and using post-operative dental pain as a model for acute pain with the following study design:
In a single-center, randomized, two-stage, parallel-group double-blind study the efficacy and safety of the fixed combination of ibuprofen 400 mg and caffeine 100 mg (in the figures abbreviated as "Ibup/Caff') was investigated in comparison with ibuprofen 400 mg, caffeine 100 mg and placebo in patients with post-operative dental pain.

| Study stage 1 | Study stage 2 | Number of patients |
|---|---|---|
| Ibuprofen + caffeine | Ibuprofen + caffeine | 210 |
| Ibuprofen | Ibuprofen | 210 |
| Caffeine | Ibuprofen + caffeine | 35 |
| Caffeine | Ibuprofen | 35 |
| Placebo | Ibuprofen + caffeine | 35 |
| Placebo | Ibuprofen | 35 |

Primary goal: Demonstration of the superior efficacy of the fixed combination of ibuprofen 400 mg and caffeine 100 mg compared with each of the individual active substances alone and compared with placebo for the treatment of post-operative dental pain over a period of 8 hours followed by a single dose of the medication (study stage 1).

Secondary goal: Evaluation of the efficacy and safety of multiple doses of the fixed combination compared with ibuprofen alone over a post-operative time period of 5 days (study stage 2).

Male and female patients between the ages of 18 and 55 years, scheduled for the extraction of 3 to 4 unsound wisdom teeth, with at least 2 extracted molars were recruited; the baseline of dental pain intensity must be at least moderate on a verbal evaluation scale and at least 5 on a numerical evaluation scale ranging from 0 to 10.

Following surgery, one film coated tablet every 6-8 hours for 5 days has been administered. Patients who received only placebo and caffeine were randomly switched to the ibuprofen or the ibuprofen/caffeine group after the first dose.

### Primary endpoint

The time-weighted sum of pain relief (PAR) and the difference in pain intensity (PID) from 0 to 8 hours (SPRID0-8H)

### Secondary endpoint

- The time-weighted sum of PAR and PID from 0 to 2 hours (SPRID0-2H)
- Duration of pain relief
- Time to significant pain relief

The pain intensity (PI) was evaluated in a diary before administration and at 0.25, 0.5, 0.75, 1, 1.5, 2, 3, 4, 5, 6, 7 and 8 hours after the first dose of the study medication using an 11-point numerical rating scale (NPRS) from 0 = "no pain" to 10 = "worst possible pain".

The pain relief (PAR) beginning from pain onset was evaluated in a patient diary using a 5-point rating scale (VRS) (0 = no pain relief; 1 = a little pain relief; 2 = some pain relief; 3 = much pain relief; 4 = complete pain relief) at the same time points as for the PI evaluation.

As soon as a patient needed an emergency medication or a second dose of the medication within less than 8 hours, PI and PAR were evaluated before the emergency medication or second dose was administered.

The time-weighted sum of pain relief (PAR) and the pain intensity difference (PID) relative to baseline between 0 and 8 hours is determined as follows:
SPRID0- 8h = (PID0.25+PAR0 .25+PID0. 5+PAR0. 5+PID0 .75+PAR0 .7 5+PID1+PAR1)/4 + (PID1.5+PAR1.5+PID2+PAR2)/2 + PID3+PAR3+PID4+PAR4+PID5+PAR5+PID6+PAR6+PID7+PAR7+PID8+PAR8, wherein the abbreviations PIDPID/PAR0.25/0.5/0.75/1/1.5/2/3/4/5/6/7/8 represent PID/PAR values at the times of 0.25, 0.5, 0.75, 1, 1.5, 2, 3, 4, 5, 6, 7 and 8 hours respectively

PID = PI at baseline - PI at the specific time point (here, higher PID values represent greater benefit for the patient).

Higher values of the SPRID0-8h likewise indicate greater benefit for the patient.

A total of 70 patients were treated with placebo or caffeine, 279 patients with ibuprofen (1 patient did not participate in step II of the trial) and 282 patients with the combination.

Table I below gives the respective mean SPRID0-8h value for the various treatments:

**Table I: Adjusted Mean SPRID0-8h value**

| Treatment | Placebo | Caffeine | Ibuprofen | Ibuprofen + caffeine |
|---|---|---|---|---|
| No. of patients | 70 | 70 | 209 | 213 |
| SPRID0-8h | 10.554 | 15.824 | 40.165 | 52.291 |

The superiority of the combination over both individual therapies and placebo was demonstrated.

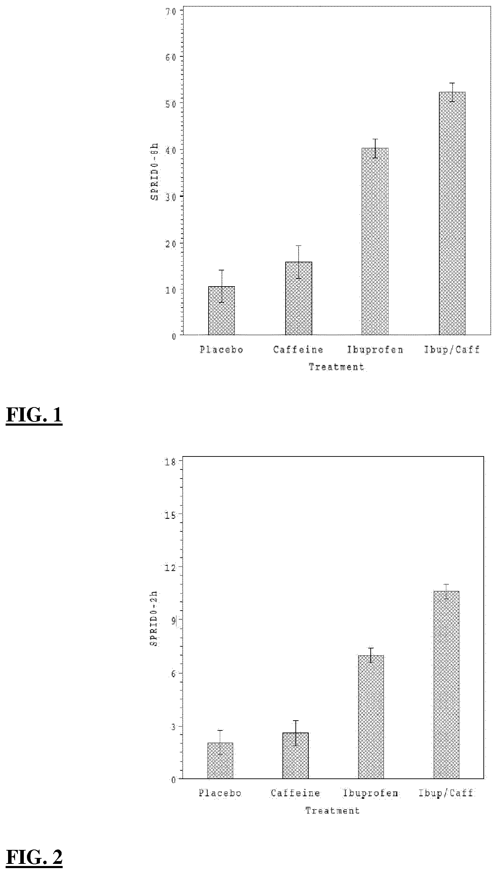

The combination of ibuprofen 400 mg and caffeine 100 mg demonstrated statistically significant superiority in terms of the primary endpoint SPRID0-8h compared with both individual treatments and placebo (cp. Fig. 1).

The efficacy findings are in line with the results of a meta-analysis on caffeine as a co-analgesic (Derry CJ, Derry S, Moore RA: Caffeine as an analgesic adjuvant for acute pain in adults (review), Cochrane Database Syst Rev (12),, CD009281 (2014)). Interestingly, ibuprofen as analgesic shows a "ceiling effect", i.e. single doses higher than 400 mg do not provide additional analgesia (Laska EM, Sunshine A, Marrero I, Olson N, Siegel C, McCormick N: The correlation between blood levels of ibuprofen and clinical analgesic response; Clin Pharmacol Ther 40 (1), 1 - 7 (1986); Kellstein DE, Lipton RB, Geetha R, Koronkiewicz K, Evans FT, Stewart WF, Wilkes K, Furey SA, Subramanian T, Cooper SA: Evaluation of a novel solubilized formulation of ibuprofen in the treatment of migraine headache: a randomized, double-blind, placebo-controlled, dose-ranging study; Cephalalgia 20, 233 - 243 (2000)). Thus, adding 100 mg caffeine to an ibuprofen dose of 400 mg provides more analgesic efficacy than expected for higher doses of ibuprofen alone.

The results of the primary endpoint were supported by the secondary endpoint SPRID0-2h.

Table II below gives the respectively achieved mean SPRID0-2h value for the various treatments (cp. Fig. 2):

**Table II: Adjusted Mean SPRID0-2h value**

| Treatment | Placebo | Caffeine | Ibuprofen | Ibuprofen + caffeine |
|---|---|---|---|---|
| No. of patients | 70 | 70 | 209 | 213 |
| SPRID0-2h | 2.059 | 2.612 | 6.990 | 10.584 |

Table III below presents the median duration of action achieved according to Kaplan-Meyer analysis for the various treatments:

**Table III: Median duration of action**

| Treatment | Placebo | Caffeine | Ibuprofen | Ibuprofen + caffeine |
|---|---|---|---|---|
| No. of patients | 70 | 70 | 209 | 213 |
| Duration of action [hours] | 1.6 | 2.1 | 7.1 | 7.3 |

The combination according to the invention gave the longest duration of pain relief, followed by ibuprofen, caffeine and placebo.

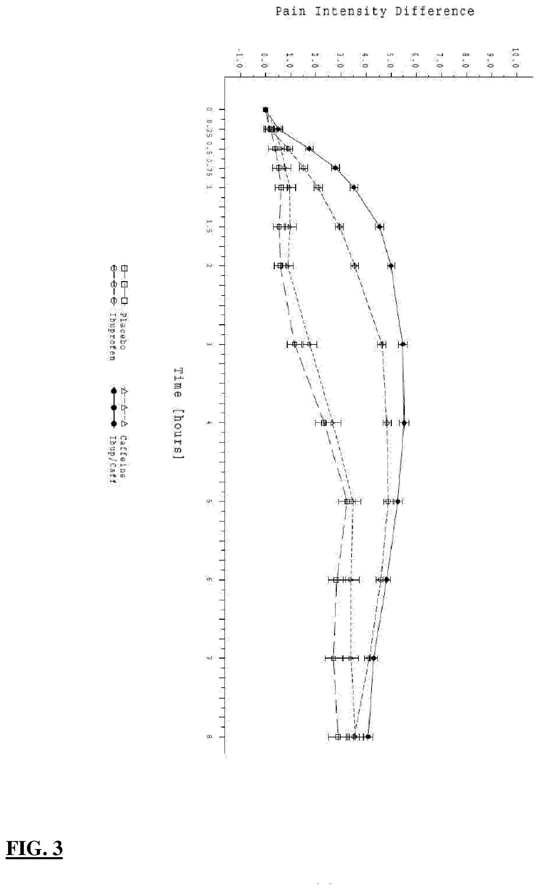

In addition, the analysis of pain intensity difference (as measured on the 0 to 10 numerical pain rating scale - NPRS) at individual time points corroborated the findings of the primary and secondary endpoint analyses. Treatment with ibuprofen/caffeine showed maintained analgesic efficacy with a fast onset, as demonstrated in the pairwise comparisons of adjusted mean pain intensities versus placebo, caffeine, and ibuprofen at individual time points. The comparison of ibuprofen/caffeine versus ibuprofen achieved statistical significance already after 0.5 h and up to 4 h of administration of trial medication. A reduction of ≥5.9 on the NPRS was only observed in the ibuprofen/caffeine arm and not in any of the other treatment arms at any time point. (cp. Fig. 3).

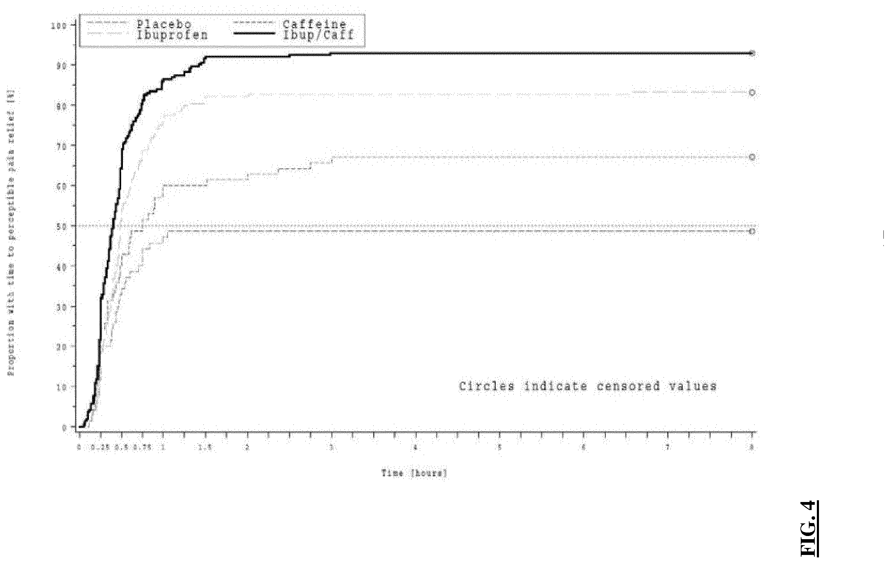

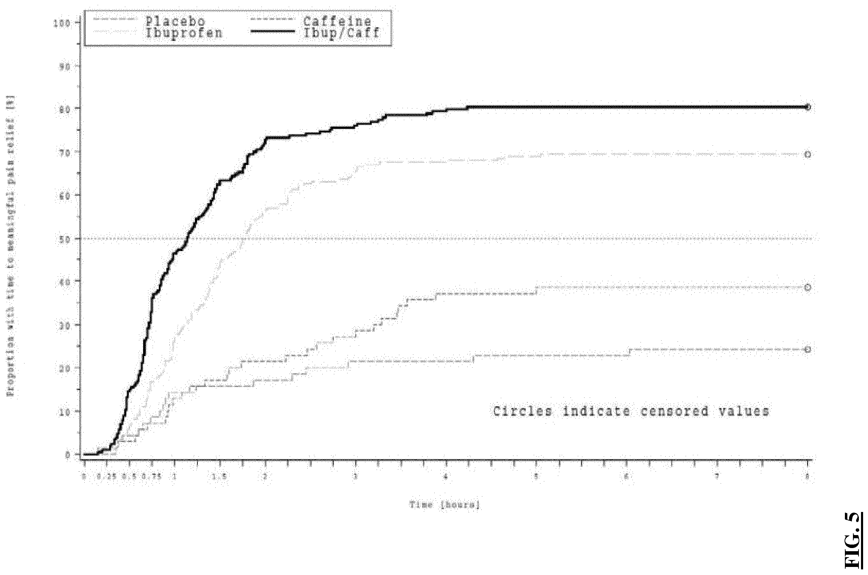

Table IV below gives the average time to significant pain relief according to Kaplan-Meyer analysis for the different treatments (cp. Figs. 4 and 5):

**Table IV: Median time to obtain relief**

| Treatment | Placebo | Caffeine | Ibuprofen | Ibuprofen + caffeine |
|---|---|---|---|---|
| No. of patients | 70 | 70 | 209 | 213 |
| Time [hours] | NC¹ | NC¹ | 1.78 | 1.13 |

| | | | | |
|---|---|---|---|---|
| ¹ NC = Not calculable (because more than half of the patients were without meaningful pain relief within 8 h) | | | | |

The combination of ibuprofen 400 mg and caffeine 100 mg demonstrated significantly shorter times to meaningful pain relief compared with both individual treatments and placebo.

### Safety:

All treatments were safe and well-tolerated.

## Claims

1. A film coated tablet wherein the core of the tablet consists of 400 mg of ibuprofen, 100 mg of caffeine, 50 to 100 mg of one or more disintegrating agents and one or more further components selected from the group of carriers, flow regulating agents and lubricants, **characterized in that**
- the core of the tablet is obtainable by direct dry compression of all the components and consists of 90 to 98 % by weight of ibuprofen, caffeine and one or more disintegrating agents and of 2 to 10% by weight of one or more further components selected from the group of carriers, flow regulating agents and lubricants;
- the disintegrating agents are selected from microcrystalline cellulose and a combination of microcrystalline cellulose and croscarmellose;
- the carrier is selected from the group consisting of lactose, cellulose, saccharose, polyethylene glycol and polyethylene oxide (PEO).

2. A tablet according to claim 1, **characterized in that** the carrier is a mixture of different types of cellulose.

3. A tablet according to claim 1 or 2, **characterized in that** the core of the tablet comprises
• 10 to 50 mg of one or more carrier materials,
• 50 to 90 mg of one or more disintegrating agents,
• 1 to 5 mg of one or more flow regulating agents, and
• 1 to 5 mg of one or more lubricants.

4. A tablet according to one of the claims 1 to 3 for use in the treatment of acute pain.

5. A process for the manufacture of a film coated tablet in accordance with any one of the claims 1 to 3, **characterized in that** a mixture consisting of 400 mg of ibuprofen, 100 mg of caffeine, 50 to 100 mg of one or more disintegrating agents and one or more further components selected from the group of carriers, flow regulating agents and lubricants is dry compressed and coated with a coating.

## Patentansprüche

1. Tablette, welche mit einem Film beschichtet ist, wobei der Kern der Tablette aus 400 mg Ibuprofen, 100 mg Koffein, 50 bis 100 mg eines oder mehr Zerfallsmittel und einem oder mehr weiteren Bestandteilen, ausgewählt aus der Gruppe von Trägern, Fließregulierungsmitteln und Gleitmitteln, besteht, **dadurch gekennzeichnet, dass**
- der Kern der Tablette durch direkte Trockenpressung aller Bestandteile erhältlich ist und aus 90-98 Gew.-% Ibuprofen, Koffein und einem oder mehr Zerfallsmitteln besteht und aus 2-10 Gew.-% eines oder mehr weiteren Bestandteilen besteht, ausgewählt aus der Gruppe von Trägern, Fließregulierungsmitteln und Gleitmitteln;
- die Zerfallsmittel ausgewählt sind aus mikrokristalliner Cellulose und einer Kombination von mikrokristalliner Cellulose und Croscarmellose;
- der Träger ausgewählt ist aus der Gruppe, bestehend aus Lactose, Cellulose, Saccharose, Polyethylenglycol und Polyethylenoxid (PEO).

2. Tablette nach Anspruch 1, **dadurch gekennzeichnet, dass** der Träger eine Mischung aus verschiedenen Arten von Cellulose ist.

3. Tablette nach Anspruch 1 oder 2, **dadurch gekennzeichnet, dass** der Kern der Tablette umfasst:
• 10 bis 50 mg eines oder mehr Trägermaterialien,
• 50 bis 90 mg eines oder mehr Zerfallsmitteln,
• 1 bis 5 mg eines oder mehr Fließregulierungsmitteln, und
• 1 bis 5 mg eines oder mehr Gleitmitteln.

4. Tablette nach einem der Ansprüche 1 bis 3 zur Verwendung bei der Behandlung von akuten Schmerzen.

5. Verfahren zur Herstellung einer Tablette, welche mit einem Film beschichtet ist, nach einem der Ansprüche 1 bis 3, **dadurch gekennzeichnet, dass** ein Gemisch, bestehend aus 400 mg Ibuprofen, 100 mg Coffein, 50 bis 100 mg eines oder mehr Zerfallsmitteln und einem oder mehr weiteren Bestandteilen, ausgewählt aus der Gruppe von Trägern, Fließregulierungsmitteln und Gleitmitteln, trocken gepresst und mit einer Beschichtung beschichtet wird.

## Revendications

1. Comprimé pelliculé dans lequel le noyau du comprimé consiste en 400 mg d'ibuprofène, 100 mg de caféine, 50 à 100 mg d'un ou plusieurs agents désintégrants et un ou plusieurs autres composants choisis dans le groupe des supports, des agents régulateurs d'écoulement et des lubrifiants, **caractérisé en ce que**
- le noyau du comprimé peut être obtenu par compression directe à sec de tous les composants et consiste en 90 à 98 % en poids d'ibuprofène, de caféine et d'un ou plusieurs agents désintégrants et en 2 à 10 % en poids d'un ou plusieurs autres composants choisis dans le groupe des supports, des agents régulateurs d'écoulement et des lubrifiants;
- les agents désintégrants sont choisis parmi la cellulose microcristalline et une combinaison de cellulose microcristalline et de croscarmellose;
- le support est choisi dans le groupe consistant en le lactose, la cellulose, le saccharose, le polyéthylène glycol et le poly(oxyde d'éthylène) (PEO).

2. Comprimé selon la revendication 1, **caractérisé en ce que** le support est un mélange de différents types de cellulose.

3. Comprimé selon la revendication 1 ou 2, **caractérisé en ce que** le noyau du comprimé comprend
• 10 à 50 mg d'un ou plusieurs matériaux supports,
• 50 à 90 mg d'un ou plusieurs agents désintégrants,
• 1 à 5 mg d'un ou plusieurs agents régulateurs d'écoulement, et
• 1 à 5 mg d'un ou plusieurs lubrifiants.

4. Comprimé selon l'une des revendications 1 à 3 destiné à être utilisé dans le traitement de la douleur aiguë.

5. Procédé pour la fabrication d'un comprimé pelliculé selon l'une quelconque des revendications 1 à 3 **caractérisé en ce qu'**un mélange consistant en 400 mg d'ibuprofène, 100 mg de caféine, 50 à 100 mg d'un ou plusieurs agents désintégrants et un ou plusieurs autres composants choisis dans le groupe des supports, des agents régulateurs d'écoulement et des lubrifiants est comprimé à sec et enrobé avec un enrobage.
